# EUROPÄISCHE PATENTSCHRIFT

(11) **EP 0 548 194 B1**
(45) Veröffentlichungstag und Bekanntmachung des Hinweises auf die Patenterteilung: **24.05.1995**
(21) Anmeldenummer: 91916474.9
(22) Anmeldetag: 12.09.1991
(51) Int. Cl.: A61K 39/395

(54) **MONOKLONALE ANTIKÖRPER GEGEN DEN INTERLEUKIN 2-REZEPTOR**
MONOCLONAL ANTIBODY ACTIVE AGAINST THE INTERLEUKIN-2 RECEPTOR
ANTICORPS MONOCLONAUX DU RECEPTEUR D'INTERLEUKINE-2

(30) Priorität: 12.09.1990 DE 4028955
(43) Veröffentlichungstag der Anmeldung: 30.06.1993
(73) Patentinhaber: BOEHRINGER MANNHEIM GMBH, 68298 Mannheim (DE)
(72) Erfinder: WEIDLE, Ulrich, D-8000 München 2 (DE); RUSSMANN, Eberhard, D-8122 Penzberg (DE); HIRTH, Klaus-Peter, D-8000 München 70 (DE); DIAMANTSTEIN, Tiberiu, D-1000 Berlin 19 (DE); KALUZA, Brigitte, D-8173 Bad Heilbrunn (DE)
(74) Vertreter: Huber, Bernhard, Dipl.-Chem.
(86) Internationale Anmeldenummer: EP9101737
(87) Internationale Veröffentlichungsnummer: WO9204051

(56) Entgegenhaltungen:
- EP-A- 241 811
- EP-A- 421 876
- WO-A-88/09671
- WO-A-89/09622
- FR-A- 2 652 747

## Beschreibung

Bei der Antwort des Immunsystems auf ein Antigen kommt es zur Aktivierung verschiedener Zellen des Immunsystems. Diese aktivierten Zellen synthetisieren eine Reihe von Lymphokinen, die dann wiederum die Aktivierung, Proliferation und Differenzierung weiterer Zellen des Immunsystems regulieren. Eine zentrale Rolle spielen dabei die T-Lymphozyten, deren Wachstum von einem spezifischen Faktor kontrolliert wird, dem Interleukin 2 (IL-2). IL-2 ist ein Lymphokin von 133 Aminosäuren und entfaltet seine wachstumsfördernde Eigenschaft über die Bindung an spezifische Rezeptoren auf der Zelloberfläche.

Der Interleukin 2-Rezeptor (IL-2R) existiert in drei Formen, die sich in ihrer Zusammensetzung und in ihrer Affinität zu dem Liganden unterscheiden. Der niedrigaffine IL-2R (K_{d} = 10⁻⁸ M), der auch als α-Kette, leichte (L) Kette, p55 Molekül, sowie beim Menschen auch als CD25 oder als Tac-Antigen bezeichnet wird, ist ein Glycoprotein von 55 kDa Molekulargewicht. Die Gene für die α-Kette des Maus-, Rind- und menschlichen Il-2R sind kloniert und die Aminosäuresequenzen ihrer Proteinprodukte sind bestimmt (Miller et al., J.Immunol. 134 (1985) 4212-4217; Nikaido et al., Nature 311 (1984) 631; Weinberg et al., Immunology 63 (1988), 603).

Der mittelaffine IL-2R (K_{d} = 10⁻⁹ M), der auch als β-Kette, schwere (H) Kette oder p75 Molekül bezeichnet wird, ist ein Glykoprotein von 70 bis 75 kDa Molekulargewicht bei Maus und Mensch (Tsudo et al., Proc.Natl.Acad.Sci. USA 83 (1986), 9694; Sharon et al., J.Exp.Med. 167 (1988), 1265).

Der hochaffine IL-2R (K_{d} = 10⁻¹¹ M) ist ein Heterodimer, das aus nicht kovalent gebundenen α- und β-Ketten besteht (Wang und Smith, J.Exp.Med. 166 (1987), 1156; Waldman, J.Nat.Cancer Institute 81 (1989) 915).

Monoklonale Antikörper sowohl gegen die α- als auch gegen die β-Kette des humanen IL-2-Rezeptors wurden isoliert und ihre immunsuppressive Wirkung sowohl in vitro als auch in vivo gezeigt (Sakagami et al., Transplantation Proceedings Vol. XIX (1) (1987), 586-590; Kupiec-Weglinski et al., Proc.Natl. Acad.Sci. USA 83 (1986), 2624-2627; Mouzaki et al., Eur.J. Immunol. 17 (1987), 335-341; Olive et al., Eur.J.Immunol. 16 (1986), 611-616; Friend et al., Transplantation Proceedings, Vol. XIX (1987), 4317-4418; Soulillou et al., Lancet, 13. Juni (1987), 1339-1342; Kupiec-Weglinski et al., Eur.J.Immunol. 17 (1987), 313-319).

Monoklonale Antikörper gegen den IL-2R besitzen ein großes Potential für die Immunsuppressiv-Therapie, insbesondere zur Behandlung der Transplantatabstoßungen, der Erwachsenen-T-Zell-Leukämie und von Autoimmunkrankheiten wie z.B. rheumatoider Arthritis.

Es hat sich jedoch gezeigt, daß die in vivo-Verabreichung von monoklonalen Antikörpern tierischen Ursprungs beim Menschen begrenzt ist, da sie als fremd erkannt werden und dies eine Immunreaktion gegen die verabreichten Antikörper hervorruft. Diese Immunreaktion kann durch Verwendung von sogenannten chimären oder humanisierten Antikörpern verringert werden. Chimäre Antikörper sind Antikörper, bei denen die konstante Domäne tierischen Ursprungs (z.B. der Maus) durch eine menschliche konstante Domäne ersetzt wurde. Humanisierte Antikörper sind menschliche Antikörper, von denen nur die Antigen-Bindungsstellen der variablen Region (die CDR- oder hypervariablen Regionen) tierischen Ursprungs sind (Verhoeyen und Riechmann, BioEssays 8 (1988), 74-78).

Doch selbst bei Verabreichung von chimärisierten oder humanisierten Antikörpern in hohen Dosen konnte in einigen Fällen eine unerwünschte Immunreaktion des Patienten aufgrund der Erzeugung von antidiotypischen Antikörpern beobachtet werden.

Aufgabe der vorliegenden Erfindung ist daher die Bereitstellung von Antikörpern, die bei einer Immunsuppressionstherapie wirksam sind, und in einer wesentlich geringeren Dosierung eingesetzt werden können als bereits bekannte Antikörper.

Ein Gegenstand der vorliegenden Erfindung ist eine Antikörper-Zusammensetzung, welche die Bindung von Interleukin 2 an seinem hochaffinen Rezeptor hemmt und
(1) monoklonale Antikörper gegen die α-Kette des Interleukin 2 Rezeptors und
(2) monoklonale Antikörper gegen die β-Kette des Interleukin 2 Rezeptors enthält, wobei jeder der Antikörper (1) und (2) für sich alleine bereits eine Hemmung der Interleukin 2 Bindung bewirkt.

Der kombinierte Einsatz von monoklonalen Antikörpern sowohl gegen die α- als auch gegen die β-Kette des IL-2-Rezeptors in vitro führt überraschenderweise zu synergistischen Effekten, so daß durch die erfindungsgemäße Antikörperzusammensetzung eine stärkere Inhibition der IL-2-induzierten Prolifertation humaner peripherer Blutlymphozyten bewirkt wird, als es bei Einsatz eines der beiden Antikörper allein bei der entsprechenden Konzentration beobachtet wird.

Eine erfindungsgemäße Antikörperzusammensetzung enthält günstigerweise
(1) von 1 bis 99 % auf Basis der Antikörper-Gesamtmenge monoklonale Antikörper gegen die α-Kette des Interleukin 2 Rezeptors und
(2) von 99 bis 1 % auf Basis der Antikörper-Gesamtmenge monoklonale Antikörper gegen die β-Kette des Interleukin 2 Rezeptors.

Vorzugsweise enthält die Zusammensetzung 4 bis 96 % auf Basis der Antikörper-Gesamtmenge monoklonale Antikörper gegen die α-Kette des Interleukin 2 Rezeptors und 96 bis 4 % auf Basis der Antikörper-Gesamtmenge monoklonale Antikörper gegen die β-Kette des Interleukin 2 Rezeptors. Bei einem Antikörper-Verhältnis von 4:96 bzw. 96:4 wird bereits eine fünffache Reduktion in der Antikörper-Gesamtmenge gefunden als wenn man nur einen monoklonalen Antikörper gegen die α- oder die β-Kette verwendet.

Besonders bevorzugt ist die erfindungsgemäße Zusammensetzung, wenn sie etwa gleiche Mengen (1) von monoklonalen Antikörpern gegen die α-Kette des Interleukin 2 Rezeptors und (2) monoklonalen Antikörpern gegen die β-Kette des Interleukin 2 Rezeptors enthält. Bei einem äquimolaren Antikörper-Verhältnis wurde eine fünfzehnfache Reduktion der Gesamtkonzentration der monoklonalen Antikörper gefunden, die für eine 60 bis 70 %ige Hemmung der IL-2-Wirkung erforderlich ist.

Als Antikörper gegen die α-Kette, die in der erfindungsgemäßen Zusammensetzung vorhanden sind, wird vorzugsweise der Antikörper 3G10/179 (ECACC 90071905) eingesetzt. Es sind jedoch auch andere Antikörper gegen die α-Kette von Interleukin 2 geeignet, insbesondere wenn sie für sich alleine bereits eine Hemmung der Interleukin 2-Bindung bewirken. Als Antikörper gegen die β-Kette des Interleukin 2 Rezeptors ist der Antikörper C68/41 (ECACC 90090704) oder der Antikörper A23A41 (DSM ACC2015) besonders geeignet, ebenso sind jedoch aus der Literatur bekannte Antikörper, wie etwa der Mik/β₁-Antikörper (Tsudo et al., Proc.Natl.Acad.Sci. USA 86 (1989), 1982-1986; Takeshita et al., J.Exp.Med. 169 (1989), 1323-1332) geeignet.

Die erfindungsgemäße Zusammensetzung kann auch ein kovalentes Kopplungsprodukt aus einem monoklonalen Antikörper gegen die α-Kette und einem monoklonalen Antikörper gegen die β-Kette des Interleukin 2-Rezeptors enthalten.

Vorzugsweise enthält die erfindungsgemäße Zusammensetzung einen oder mehrere chimärisierte Antikörper mit humanen konstanten Domänen bzw. humanisierte Antikörper, bei denen auch die nicht hypervariablen Teile der variablen Domäne durch die entsprechenden menschlichen Regionen ersetzt sind. Dabei verwendet man günstigerweise chimärisierte Antikörper, deren variable Domänen durch neue, in DE 40 33 120 sowie einer weiteren korrespondierenden Anmeldung beschriebene Verfahren isoliert wurden.

Weiterhin beinhaltet die vorliegende Erfindung ein Arzneimittel, das eine erfindungsgemäße Antikörper-Zusammensetzung, sowie gegebenenfalls übliche pharmazeutische Träger-, Füll-, Hilfs- und Zusatzstoffe enthält, sowie ein Verfahren zur Herstellung eines solchen Arzneimittels, insbesondere zur Immunsuppressionstherapie von lymphoproliferativen Krankheiten, Autoimmunkrankheiten, Transplantatabstoßungen oder sonstigen Störungen im Organismus, bei denen die Proliferation von T-Zellen zumindest zeitweise unterdrückt werden muß. Weiterhin beinhaltet die Erfindung die Verwendung eines solchen Arzneimittels zur Immunsuppressionstherapie.

Schließlich beinhaltet die Erfindung auch ein Verfahren zur Bekämpfung von Störungen des Immunsystems, insbesondere zur Immunsuppression, worin man ein erfindungsgemäßes Arzneimittel verabreicht.

Die Zellinien, welche die genannten Antikörper produzieren, wurden bei der European Collection of Animal Cell Cultures (ECACC), Porton Down (GB) bzw. der Deutschen Sammlung von Mikroorganismen und Zellkulturen GmbH (DSM), Mascheroder Weg 1b, D-3300 Braunschweig hinterlegt und erhielten die Hinterlegungsnummern:
3G10/179 : ECACC 90071905 (Hinterlegungsdatum: 19.07.1990)
C68/41 : ECACC 90090704 (Hinterlegungsdatum: 07.09.1990)
A23A41 : DSM ACC2015 (Hinterlegungsdatum: 30.07.1991)
Die Sequenzen der variablen Regionen des Antikörpers A23A41 sind in den beiliegenden Sequenzprotokollen beschrieben. Geeignete konstante Regionen (murin oder human) für diesen Antikörper sind beschrieben in: Sequences of proteins of immunological interest; E. Kabat, T. Wu, M. Reid-Miller, H. Perry und K. Gottesman, US Department of Health and Human Services, 1987, p. 282-325.

Die Erfindung soll noch durch folgendes Beispiel in Verbindung mit den Sequenzprotokollen verdeutlicht werden.
- SEQ ID NO. 1: zeigt die Nukleotid- und Aminosäuresequenz der variablen Region der leichten Kette des Anti-IL2Rβ-Antikörpers A23A41.
- SEQ ID NO. 2: zeigt die Nukleotid- und Aminosäuresequenz der variablen Region der schweren Kette des Anti-IL2Rβ-Antikörpers A23A41.

### Beispiel 1

### 1. Präaktivierung humaner peripherer Blutlymphozyten

Periphere Blutlymphozyten werden über einen Ficoll-Gradienten angereichert, in Zellkulturmedium gewaschen und mit 5 »g/ml Concanavalin A bei einer Zelldichte von 8x10⁵ Zellen/ml für 3 Tage inkubiert (37°C, 5 % CO₂). Danach werden die Zellen mit Kulturmedium mit 5 mg/ml Methyl-α-mannopyranosid zur Entfernung des ConA gewaschen und in den Test eingesetzt.

### 2. Testdurchführung

Die präaktivierten, gewaschenen, humanen, peripheren Blutlymphozyten werden auf 4x10⁶ Zellen/ml in Kulturmedium eingestellt und je 25 »l der Zellsuspension mit je 25 »l der monoklonalen Antikörper gegen den IL-2 Rezeptor (Konzentration siehe Tabelle 1) für eine halbe Stunde bei Raumtemperatur in einer 96-Loch Mikrotiterplatte inkubiert. Danach wird 25 »l einer IL-2 Lösung (100 U/ml) zugegeben und die Kultur für 48 Stunden inkubiert (37°C, 5 % CO₂).

Anschließend werden Vitalität und Wachstum der Lymphoblasten mit Hilfe des Vitalfabstoffes 3-(4,5-Dimethyl-2-thiazolil)-2,5-diphenyltetrazoliumbromid (MTT) bestimmt. Dazu wird pro Mikrokulturansatz 10 »l einer MTT-Lösung (5mg/ml in PBS) zugegeben und bei 37°C für 4 Stunden inkubiert. Die Lösung des blauen Formazans erfolgt nach Zugabe von 100 »l pro Mikrokultur von einer 10 %igen SDS-Lösung in 0,01 N HCl bei 37°C über Nacht. Danach wird die Extinktion des umgesetzten Farbstoffs am ELISA-Reader bei einer Meßwellenlänge von 550 nm gegen eine Referenzwellenlänge von 690 nm gemessen und die Inhibition der Farbstoffumsetzung gegenüber einer Kontrolle ohne monoklonalen Antikörper gegen den IL-2 Rezeptor bestimmt.

### 3. Ergebnis

Überraschenderweise benötigt man bei einer geeigneten Kombination der beiden Antikörper eine um den Faktor 15 geringere Antikörperkonzentration als wenn man jeweils einen der beiden Antikörper alleine einsetzt (Tabelle 1).

**Tabelle 1**

| Für eine 60 bis 70 %ige Hemmung der IL-2 induzierten Proliferation präaktivierter humaner peripherer Blutlymphozyten werden an monoklonalem Antikörper gegen den IL-2 Rezeptor in vitro benötigt: | | | |
|---|---|---|---|
| anti-α MAK 3G10/179 | anti-β MAK C68/41 oder A23A41 | Gesamtkonz. MAK | Reduktion |
| 62,5 »g/ml | 0 »g/ml | 62,5 »g/ml | 0 |
| 12 »g/ml | 0,5 »g/ml | 12,5 »g/ml | 5-fach |
| 2 »g/ml | 2 »g/ml | 4 »g/ml | 15-fach |
| 0,5 »g/ml | 12 »g/ml | 12,5 »g/ml | 5-fach |
| 0 »g/ml | 62,5 »g/ml | 62,5 »g/ml | 0 |

SEQ.ID.NO.: 1
ART DER SEQUENZ: Nukleinsäure mit entsprechendem Protein
SEQUENZLÄNGE: 322 bp
MERKMALE: aa 1-96 V-Region
aa 97-107 J-Region
bp 322 erstes Basenpaar der C-Region

GAC GTC TTG CTG ACT CAG TCT CCA GCC ATC CTG TCC GTG AGT CCA GGA 48
Asp Val Leu Leu Thr Gln Ser Pro Ala Ile Leu Ser Val Ser Pro Gly
1               5                   10                  15

GAA AGA GTC AGT TTC TCC TGT AGG GCC AGT CAG AGC ATT GGC ACA AGC 96
Glu Arg Val Ser Phe Ser Cys Arg Ala Ser Gln Ser Ile Gly Thr Ser
            20                  25                  30

ATA CAC TGG TAT CAG CAA AGA ACA AAT GGT CCT CCA AGG CTT CTC ATA 144
Ile His Trp Tyr Gln Gln Arg Thr Asn Gly Pro Pro Arg Leu Leu Ile
        35                  40                  45

AAG TAT GCG TCT GAG TCA ATC TCT GGG ATC CCT TCC AGG TTT AGT GGC 192
Lys Tyr Ala Ser Glu Ser Ile Ser Gly Ile Pro Ser Arg Phe Ser Gly
    50                  55                  60

AGT GGA TCA GGG ACA GAT TTT ACT CTT AGC ATC AGC AGT GTG GAG TCT 240
Ser Gly Ser Gly Thr Asp Phe Thr Leu Ser Ile Ser Ser Val Glu Ser
65                  70                  75                  80

GAA GAT ATT GCA GAT TAT TAC TGT CAA CAA ACT AAT AGC TGG CCA ACC 288
Glu Asp Ile Ala Asp Tyr Tyr Cys Gln Gln Thr Asn Ser Trp Pro Thr
                85                  90                  95

ACG TTC GGA GGG GGG ACC AAG CTG GAA ATT AAA C 322
Thr Phe Gly Gly Gly Thr Lys Leu Glu Ile Lys
            100                 105

SEQ.ID.NO.: 2
ART DER SEQUENZ: Nukleinsäure mit entsprechendem Protein
SEQUENZLÄNGE: 355 bp
MERKMALE: aa 1-98 V-Region
          aa 99-104 D-Region
          aa 105-118 J-Region
          bp 355 erstes Basenpaar der C-Region

GAG GTC CAG CTG CAA CAG TTT GGA GCT GAA TTG GTG AAG CCT GGG ACT   48
Glu Val Gln Leu Gln Gln Phe Gly Ala Glu Leu Val Lys Pro Gly Thr
  1               5                  10                  15

TCG GTG AAG ATA TCC TGC AAG GCT TCT GGC TAC ATT TTC ACT GAC TAC   96
Ser Val Lys Ile Ser Cys Lys Ala Ser Gly Tyr Ile Phe Thr Asp Tyr
             20                  25                  30

AAC ATG GAC TGG GTG AAG CAG AGC CAT GGA AAG AGC CTT GAG TGG ATT   144
Asn Met Asp Trp Val Lys Gln Ser His Gly Lys Ser Leu Glu Trp Ile
         35                  40                  45

GGA GAT ATT GAT CCT AAC TTT GAT AGT TCC AGT TAC AAC CAG AAG TTC   192
Gly Asp Ile Asp Pro Asn Phe Asp Ser Ser Ser Tyr Asn Gln Lys Phe
     50                  55                  60

AAG GGA AAG GCC ACA TTG ACT GTA GAC AAG TCC TCC AAC ACA GCC TAC   240
Lys Gly Lys Ala Thr Leu Thr Val Asp Lys Ser Ser Asn Thr Ala Tyr
 65                  70                  75                  80

ATG GAG CTC CGC AGC CTG ACA TCT GAG GAC ACT GCA GTC TAT TAC TGT   288
Met Glu Leu Arg Ser Leu Thr Ser Glu Asp Thr Ala Val Tyr Tyr Cys
                 85                  90                  95

GCA AGA GGG GGA TTC CCC TAT GGT ATG GAC TAC TGG GGT CAA GGA ACC   336
Ala Arg Gly Gly Phe Pro Tyr Gly Met Asp Tyr Trp Gly Gln Gly Thr
            100                 105                 110

TCA GTC ACC GTC TCC TCA G  355
Ser Val Thr Val Ser Ser
        115

## Patentansprüche (Patentansprüche für folgende(n) Vertragsstaat(en): AT, BE, CH, DE, DK, FR, GB, IT, LI, LU, NL, SE)

1. Antikörper-Zusammensetzung,
**dadurch gekennzeichnet,**
daß sie die Bindung von Interleukin 2 an seinen hochaffinen Rezeptor hemmt und
(1) monoklonale Antikörper gegen die α-Kette des Interleukin 2 Rezeptors und
(2) monoklonale Antikörper gegen die β-Kette des Interleukin 2 Rezeptors enthält, wobei jeder der Antikörper (1) und (2) für sich alleine bereits eine Hemmung der Interleukin 2 Bindung bewirkt.

2. Zusammensetzung nach Anspruch 1,
**dadurch gekennzeichnet,**
daß sie
(1) von 1 bis 99 % auf Basis der Antikörper-Gesamtmenge monoklonale Antikörper gegen die α-Kette des Interleukin 2 Rezeptors und
(2) von 99 bis 1 % auf Basis der Antikörper-Gesamtmenge monoklonale Antikörper gegen die β-Kette des Interleukin 2 Rezeptors enthält.

3. Zusammensetzung nach Anspruch 1 oder 2,
**dadurch gekennzeichnet,**
daß sie
(1) von 4 bis 96 % auf Basis der Antikörper-Gesamtmenge monoklonale Antikörper gegen die α-Kette des Interleukin 2 Rezeptors und
(2) von 96 bis 4 % auf Basis der Antikörper-Gesamtmenge monoklonale Antikörper gegen die β-Kette des Interleukin 2 Rezeptors enthält.

4. Zusammensetzung nach Anspruch 3,
**dadurch gekennzeichnet,**
daß etwa gleiche Mengen (1) von monoklonalen Antikörpern gegen die α-Kette des Interleukin 2 Rezeptors und (2) monoklonalen Antikorpern gegen die β-Kette des Interleukin 2 Rezeptors enthält.

5. Zusammensetzung nach einem der Ansprüche 1 bis 4,
**dadurch gekennzeichnet,**
daß sie den Antikörper 3G10/179 (ECACC 90071905) gegen die α-Kette des Interleukin 2 Rezeptors enthält.

6. Zusammensetzung nach einem der Ansprüche 1 bis 4,
**dadurch gekennzeichnet,**
daß sie den Antikörper C68/41 (ECACC 90090704) gegen die β-Kette des Interleukin 2 Rezeptors enthält.

7. Zusammensetzung nach einem der Ansprüche 1 bis 4,
**dadurch gekennzeichnet,**
daß sie den Antikörper A23A41 (DSM ACC2015) gegen die β-Kette des Interleukin 2 Rezeptors enthält.

8. Zusammensetzung nach einem der vorhergehenden Ansprüche,
**dadurch gekennzeichnet,**
daß sie ein kovalentes Kopplungsprodukt aus einem monoklonalen Antikörper gegen die α-Kette und einem monoklonalen Antikörper gegen die β-Kette des Interleukin 2 Rezeptors enthält.

9. Zusammensetzung nach einem der vorhergehenden Ansprüche,
**dadurch gekennzeichnet,**
daß sie chimärisierte Antikörper mit humanen konstanten Domänen enthält.

10. Zusammensetzung nach Anspruch 9,
**dadurch gekennzeichnet,**
daß sie humanisierte Antikörper enthält.

11. Arzneimittel,
**dadurch gekennzeichnet,**
daß es eine Antikörper-Zusammensetzung nach einem der Ansprüche 1 bis 10, sowie gegebenenfalls übliche pharmazeutische Träger-, Füll-, Hilfs- und Zusatzstoffe enthält.

12. Verfahren zur Herstellung eines Arzneimittels zur Immunsuppressionstherapie ,
**dadurch gekennzeichnet,**
daß man eine Antikörper-Zusammensetzung nach einem der Ansprüche 1 bis 10, gegebenenfalls mit üblichen pharmazeutischen Träger-, Füll-, Hilfs- und Zusatzstoffen formuliert.

13. Verfahren zur Herstellung eines Arzneimittels zur Bekämpfung von Störungen des Immunsystems,
**dadurch gekennzeichnet,**
daß man eine Antikörper-Zusammensetzung nach einem der Ansprüche 1 bis 10, gegebenenfalls mit üblichen pharmazeutischen Träger-, Füll-, Hilfs- und Zusatzstoffen formuliert.

14. Verfahren zur Herstellung eines Arzneimittels zur Unterdrückung der Proliferation von T-Zellen,
**dadurch gekennzeichnet,**
daß man eine Antikörper-Zusammensetzung nach einem der Ansprüche 1 bis 10, gegebenenfalls mit üblichen pharmazeutischen Träger-, Füll-, Hilfs- und Zusatzstoffen formuliert.

## Patentansprüche (Patentansprüche für folgende(n) Vertragsstaat(en): GR, ES)

1. Verfahren zur Herstellung einer Antikörper-Zusammensetzung, welche die Bindung von Interleukin 2 an seinen hochaffinen Rezeptor hemmt,
**dadurch gekennzeichnet,**
daß man
(1) monoklonale Antikörper gegen die α-Kette des Interleukin 2 Rezeptors und
(2) monoklonale Antikörper gegen die β-Kette des Interleukin 2 Rezeptors gemeinsam formuliert, wobei jeder der Antikörper (1) und (2) für sich alleine bereits eine Hemmung der Interleukin 2 Bindung bewirkt.

2. Verfahren nach Anspruch 1,
**dadurch gekennzeichnet,**
daß die Zusammensetzung
(1) von 1 bis 99 % auf Basis der Antikörper-Gesamtmenge monoklonale Antikörper gegen die α-Kette des Interleukin 2 Rezeptors und
(2) von 99 bis 1 % auf Basis der Antikörper-Gesamtmenge monoklonale Anitkörper gegen die β-Kette des Interleukin 2 Rezeptors enthält.

3. Verfahren nach Anspruch 1 oder 2,
**dadurch gekennzeichnet,**
daß die Zusammensetzung
(1) von 4 bis 96 % auf Basis der Antikörper-Gesamtmenge monoklonale Antikörper gegen die α-Kette des Interleukin 2 Rezeptors und
(2) von 96 bis 4 % auf Basis der Antikörper-Gesamtmenge monoklonale Antikörper gegen die β-Kette des Interleukin 2 Rezeptors enthält.

4. Verfahren nach Anspruch 3,
**dadurch gekennzeichnet,**
daß die Zusammensetzung etwa gleiche Mengen von (1) monoklonalen Antikörpern gegen die α-Kette des Interleukin 2 Rezeptors und (2) monoklonalen Antikörpern gegen die β-Kette des Interleukin 2 Rezeptors enthält.

5. Verfahren nach einem der Ansprüche 1 bis 4,
**dadurch gekennzeichnet,**
daß die Zusammensetzung den Antikörper 3G10/179 (ECACC 90071905) gegen die α-Kette des Interleukin 2 Rezeptors enthält.

6. Verfahren nach einem der Ansprüche 1 bis 4,
**dadurch gekennzeichnet,**
daß die Zusammensetzung den Antikörper C68/41 (ECACC 90090704) gegen die β-Kette des Interleukin 2 Rezeptors enthält.

7. Verfahren nach einem der Ansprüche 1 bis 4,
**dadurch gekennzeichnet,**
daß die Zusammensetzung den Antikörper A23A41 (DSM ACC2015) gegen die β-Kette des Interleukin 2 Rezeptors enthält.

8. Verfahren nach einem der vorhergehenden Ansprüche,
**dadurch gekennzeichnet,**
daß die Zusammensetzung ein kovalentes Kopplungsprodukt aus einem monoklonalen Antikörper gegen die α-Kette und einem monoklonalen Antikörper gegen die β-Kette des Interleukin 2 Rezeptors enthält.

9. Verfahren nach einem der vorhergehenden Ansprüche,
**dadurch gekennzeichnet,**
daß die Zusammensetzung chimärisierte Antikörper mit humanen konstanten Domänen enthält.

10. Verfahren nach Anspruch 9,
**dadurch gekennzeichnet,**
daß die Zusammensetzung humanisierte Antikörper enthält.

11. Verfahren zur Herstellung eines Arzneimittels,
**dadurch gekennzeichnet,**
daß man eine Antikörper-Zusammensetzung, die nach einem der Ansprüche 1 bis 10 hergestellt wurde, gegebenenfalls mit üblichen pharmazeutischen Träger-, Füll-, Hilfs- und Zusatzstoffen formuliert.

12. Verfahren zur Herstellung eines Arzneimittels zur Immunsuppressionstherapie,
**dadurch gekennzeichnet,**
daß man eine Antikörper-Zusammensetzung, die nach einem der Ansprüche 1 bis 10 hergestellt wurde, gegebenenfalls mit üblichen pharmazeutischen Träger-, Füll-, Hilfs- und Zusatzstoffen formuliert.

13. Verfahren zur Herstellung eines Arzneimittels zur Bekämpfung von Störungen des Immunsystems,
**dadurch gekennzeichnet,**
daß man eine Antikörper-Zusammensetzung, die nach einem der Ansprüche 1 bis 10 hergestellt wurde, gegebenenfalls mit üblichen pharmazeutischen Träger-, Füll-, Hilfs- und Zusatzstoffen formuliert.

14. Verfahren zur Herstellung eines Arzneimittels zur Unterdrückung der Proliferation von T-Zellen,
**dadurch gekennzeichnet,**
daß man eine Antikörper-Zusammensetzung, die nach einem der Ansprüche 1 bis 10 hergestellt wurde, gegebenenfalls mit üblichen pharmazeutischen Träger-, Füll-, Hilfs- und Zusatzstoffen formuliert.

## Claims (Claims for the following Contracting State(s): AT, BE, CH, DE, DK, FR, GB, IT, LI, LU, NL, SE)

1. Antibody composition,
**wherein**
it inhibits the binding of interleukin 2 to its high affinity receptor and contains
(1) monoclonal antibodies against the α chain of the interleukin 2 receptor and
(2) monoclonal antibodies against the β chain of the interleukin 2 receptor, wherein each of the antibodies (1) and (2) by itself already causes an inhibition of the interleukin 2 binding.

2. Composition as claimed in claim 1,
**wherein**
it contains
(1) 1 to 99 % in relation to the total antibody amount of monoclonal antibodies against the α chain of the interleukin 2 receptor and
(2) 99 to 1 % in relation to the total antibody amount of monoclonal antibodies against the β chain of the interleukin 2 receptor.

3. Composition as claimed in claim 1 or 2,
**wherein**
it contains
(1) 4 to 96 % in relation to the total antibody amount of monoclonal antibodies against the α chain of the interleukin 2 receptor and
(2) 96 to 4 % in relation to the total antibody amount of monoclonal antibodies against the β chain of the interleukin 2 receptor.

4. Composition as claimed in claim 3,
**wherein**
it contains approximately equal amounts (1) of monoclonal antibodies against the α chain of the interleukin 2 receptor and (2) of monoclonal antibodies against the β chain of the interleukin 2 receptor.

5. Composition as claimed in one of the claims 1 to 4,
**wherein**
it contains the antibody 3G10/179 (ECACC 90071905) against the α chain of the interleukin 2 receptor.

6. Composition as claimed in one of the claims 1 to 4,
**wherein**
it contains the antibody C68/41 (ECACC 90090704) against the β chain of the interleukin 2 receptor.

7. Composition as claimed in one of the claims 1 to 4,
**wherein**
it contains the antibody A23A41 (DSM ACC2015) against the β chain of the interleukin 2 receptor.

8. Composition as claimed in one of the previous claims,
**wherein**
it contains a covalent coupling product of a monoclonal antibody against the α chain and of a monoclonal antibody against the β chain of the interleukin 2 receptor.

9. Composition as claimed in one of the previous claims,
**wherein**
it contains chimerized antibodies with human constant domains.

10. Composition as claimed in claim 9,
**wherein**
it contains humanized antibodies.

11. Pharmaceutical agent,
**wherein**
it contains an antibody composition as claimed in one of the claims 1 to 10 as well as, if desired, the usual pharmaceutical carrier substances, fillers, auxiliary agents and additives.

12. Process for the production of a pharmaceutical agent for immunosuppressive therapy,
**wherein**
an antibody composition as claimed in one of the claims 1 to 10 is formulated together, if desired, with the usual pharmaceutical carrier substances, fillers, auxiliary agents and additives.

13. Process for the production of a pharmaceutical agent for treating disorders of the immune system,
**wherein**
an antibody composition as claimed in one of the claims 1 to 10 is formulated together, if desired, with the usual pharmaceutical carrier substances, fillers, auxiliary agents and additives.

14. Process for the production of a pharmaceutical agent to suppress the proliferation of T cells,
**wherein**
an antibody composition as claimed in one of the claims 1 to 10 is formulated together, if desired, with the usual pharmaceutical carrier substances, fillers, auxiliary agents and additives.

## Claims (Claims for the following Contracting State(s): ES, GR)

1. Process for the production of an antibody composition which inhibits the binding of interleukin 2 to its high affinity receptor,
**wherein**
(1) monoclonal antibodies against the α chain of the interleukin 2 receptor and
(2) monoclonal antibodies against the β chain of the interleukin 2 receptor are formulated together, wherein each of the antibodies (1) and (2) by itself already causes an inhibition of the interleukin 2 binding.

2. Process as claimed in claim 1,
**wherein**
the composition contains
(1) 1 to 99 % in relation to the total antibody amount of monoclonal antibodies against the α chain of the interleukin 2 receptor and
(2) 99 to 1 % in relation to the total antibody amount of monoclonal antibodies against the β chain of the interleukin 2 receptor.

3. Process as claimed in claim 1 or 2,
**wherein**
the composition contains
(1) 4 to 96 % in relation to the total antibody amount of monoclonal antibodies against the α chain of the interleukin 2 receptor and
(2) 96 to 4 % in relation to the total antibody amount of monoclonal antibodies against the β chain of the interleukin 2 receptor.

4. Process as claimed in claim 3,
**wherein**
the composition contains approximately equal amounts of (1) monoclonal antibodies against the α chain of the interleukin 2 receptor and (2) monoclonal antibodies against the β chain of the interleukin 2 receptor.

5. Process as claimed in one of the claims 1 to 4,
**wherein**
the composition contains the antibody 3G10/179 (ECACC 90071905) against the α chain of the interleukin 2 receptor.

6. Process as claimed in one of the claims 1 to 4,
**wherein**
the composition contains the antibody C68/41 (ECACC 90090704) against the β chain of the interleukin 2 receptor.

7. Process as claimed in one of the claims 1 to 4,
**wherein**
the composition contains the antibody A23A41 (DSM ACC2015) against the β chain of the interleukin 2 receptor.

8. Process as claimed in one of the previous claims,
**wherein**
the composition contains a covalent coupling product of a monoclonal antibody against the α chain and of a monoclonal antibody against the β chain of the interleukin 2 receptor.

9. Process as claimed in one of the previous claims,
**wherein**
the composition contains chimerized antibodies with human constant domains.

10. Process as claimed in claim 9,
**wherein**
the composition contains humanized antibodies.

11. Process for the production of a pharmaceutical agent,
**wherein**
an antibody composition that has been produced according to one of the claims 1 to 10 is formulated, if desired, together with the usual pharmaceutical carrier substances, fillers, auxiliary agents and additives.

12. Process for the production of a pharmaceutical agent for immunosuppressive therapy,
**wherein**
an antibody composition that has been produced according to one of the claims 1 to 10 is formulated, if desired, together with the usual pharmaceutical carrier substances, fillers, auxiliary agents and additives.

13. Process for the production of a pharmaceutical agent for treating disorders of the immune system,
**wherein**
an antibody composition that has been produced according to one of the claims 1 to 10 is formulated, if desired, together with the usual pharmaceutical carrier substances, fillers, auxiliary agents and additives.

14. Process for the production of a pharmaceutical agent to suppress the proliferation of T cells,
**wherein**
an antibody composition that has been produced according to one of the claims 1 to 10 is formulated, if desired, together with the usual pharmaceutical carrier substances, fillers, auxiliary agents and additives.

## Revendications (Revendications pour l'(les) Etat(s) contractant(s) suivant(s): AT, BE, CH, DE, DK, FR, GB, IT, LI, LU, NL, SE)

1. Composition d'anticorps caractérisée en ce qu'elle inhibe la liaison de l'interleukine 2 à son récepteur à haute affinité et en ce qu'elle contient
(1) des anticorps monoclonaux contre la chaîne α du récepteur de l'interleukine 2 et
(2) des anticorps monoclonaux contre la chaîne β du récepteur de l'interleukine 2, chacun des anticorps (1) et (2) provoquant déjà par lui-même une inhibition de la liaison de l'interleukine 2.

2. Composition selon la revendication 1, caractérisée en ce qu'elle contient
(1) de 1 à 99% par rapport à la quantité totale d'anticorps d'anticorps monoclonaux contre la chaîne α du récepteur de l'interleukine 2 et
(2) de 99 à 1% par rapport à la quantité totale d'anticorps d'anticorps monoclonaux contre la chaîne β du récepteur de l'interleukine 2.

3. Composition selon la revendication 1 ou 2, caractérisée en ce qu'elle contient
(1) de 4 à 96% par rapport à la quantité totale d'anticorps d'anticorps monoclonaux contre la chaîne α du récepteur de l'interleukine 2 et
(2) de 96 à 4% par rapport à la quantité totale d'anticorps d'anticorps monoclonaux contre la chaîne β du récepteur de l'interleukine 2.

4. Composition selon la revendication 3, caractérisée en ce qu'elle contient des quantités sensiblement identiques (1) d'anticorps monoclonaux contre la chaîne α du récepteur de l'interleukine 2 et (2) d'anticorps monoclonaux contre la chaîne β du récepteur de l'interleukine 2.

5. Composition selon l'une des revendications 1 à 4, caractérisée en ce qu'elle contient l'anticorps 3G10/179 (ECACC 90071905) contre la chaîne α du récepteur de l'interleukine 2.

6. Composition selon l'une des revendications 1 à 4, caractérisée en ce qu'elle contient l'anticorps C68/41 (ECACC 90090704) contre la chaîne β du récepteur de l'interleukine 2.

7. Composition selon l'une des revendications 1 à 4, caractérisée en ce qu'elle contient l'anticorps A23A41 (DSM ACC2015) contre la chaîne β du récepteur de l'interleukine 2.

8. Composition selon l'une des revendications précédentes, caractérisée en ce qu'elle contient un produit de couplage covalent constitué par un anticorps monoclonal contre la chaîne α et par un anticorps monoclonal contre la chaîne β du récepteur de l'interleukine 2.

9. Composition selon l'une des revendications précédentes, caractérisée en ce qu'elle contient des anticorps chimérisés à domaines constants humains.

10. Composition selon la revendication 9, caractérisée en ce qu'elle contient des anticorps humanisés.

11. Médicament caractérisé en ce qu'il contient une composition d'anticorps selon l'une des revendications 1 à 10 et éventuellement des véhicules, charges, adjuvants et additifs pharmaceutiques habituels.

12. Procédé de préparation d'un médicament pour la thérapie d'immunosuppression, caractérisé en ce que l'on formule une composition d'anticorps selon l'une des revendications 1 à 10, éventuellement avec des véhicules, charges, adjuvants et additifs pharmaceutiques habituels.

13. Procédé de préparation d'un médicament pour lutter contre les troubles du système immunitaire, caractérisé en ce que l'on formule une composition d'anticorps selon l'une des revendications 1 à 10, éventuellement avec des véhicules, charges, adjuvants et additifs pharmaceutiques habituels.

14. Procédé de préparation d'un médicament pour réprimer la prolifération des cellules T, caractérisé en ce que l'on formule une composition d'anticorps selon l'une des revendications 1 à 10, éventuellement avec des véhicules, charges, adjuvants et additifs pharmaceutiques habituels.

## Revendications (Revendications pour l'(les) Etat(s) contractant(s) suivant(s): ES, GR)

1. Procédé de préparation d'une composition d'anticorps qui inhibe la liaison de l'interleukine 2 à son récepteur à haute affinité caractérisé en ce que l'on formule ensemble
(1) des anticorps monoclonaux contre la chaîne α du récepteur de l'interleukine 2 et
(2) des anticorps monoclonaux contre la chaîne β du récepteur de l'interleukine 2, chacun des anticorps (1) et (2) provoquant déjà par lui-même une inhibition de la liaison de l'interleukine 2.

2. Procédé selon la revendication 1, caractérisé en ce que la composition contient
(1) de 1 à 99% par rapport à la quantité totale d'anticorps d'anticorps monoclonaux contre la chaîne α du récepteur de l'interleukine 2 et
(2) de 99 à 1% par rapport à la quantité totale d'anticorps d'anticorps monoclonaux contre la chaîne β du récepteur de l'interleukine 2.

3. Procédé selon la revendication 1 ou 2, caractérisé en ce que la composition contient
(1) de 4 à 96% par rapport à la quantité totale d'anticorps d'anticorps monoclonaux contre la chaîne α du récepteur de l'interleukine 2 et
(2) de 96 à 4% par rapport à la quantité totale d'anticorps d'anticorps monoclonaux contre la chaîne β du récepteur de l'interleukine 2.

4. Procédé selon la revendication 3, caractérisé en ce que la composition contient des quantités sensiblement identiques (1) d'anticorps monoclonaux contre la chaîne α du récepteur de l'interleukine 2 et (2) d'anticorps monoclonaux contre la chaîne β du récepteur de l'interleukine 2.

5. Procédé selon l'une des revendications 1 à 4, caractérisé en ce que la composition contient l'anticorps 3G10/179 (ECACC 90071905) contre la chaîne α du récepteur de l'interleukine 2.

6. Procédé selon l'une des revendications 1 à 4, caractérisé en ce que la composition contient l'anticorps C68/41 (ECACC 90090704) contre la chaîne β du récepteur de l'interleukine 2.

7. Procédé selon l'une des revendications 1 à 4, caractérisé en ce que la composition contient l'anticorps A23A41 (DSM ACC2015) contre la chaîne β du récepteur de l'interleukine 2.

8. Procédé selon l'une des revendications précédentes, caractérisé en ce que la composition contient un produit de couplage covalent constitué par un anticorps monoclonal contre la chaîne α et par un anticorps monoclonal contre la chaîne β du récepteur de l'interleukine 2.

9. Procédé selon l'une des revendications précédentes, caractérisé en ce que la composition contient des anticorps chimérisés à domaines constants humains.

10. Procédé selon la revendication 9, caractérisé en ce que la composition contient des anticorps humanisés.

11. Procédé de préparation d'un médicament, caractérisé en ce que l'on formule une composition d'anticorps qui a été préparée selon l'une des revendications 1 a 10, éventuellement avec des véhicules, charges, adjuvants et additifs pharmaceutiques habituels.

12. Procédé de préparation d'un médicament pour la thérapie d'immunosuppression, caractérisé en ce que l'on formule une composition d'anticorps qui a été préparée selon l'une des revendications 1 à 10, éventuellement avec des véhicules, charges, adjuvants et additifs pharmaceutiques habituels.

13. Procédé de préparation d'un médicament pour lutter contre les troubles du système immunitaire, caractérisé en ce que l'on formule une composition d'anticorps qui a été préparée selon l'une des revendications 1 à 10, éventuellement avec des véhicules, charges, adjuvants et additifs pharmaceutiques habituels.

14. Procédé de préparation d'un médicament pour réprimer la prolifération des cellules T, caractérisé en ce que l'on formule une composition d'anticorps qui a été préparée selon l'une des revendications 1 à 10, éventuellement avec des véhicules, charges, adjuvants et additifs pharmaceutiques habituels.
